(19) Europäisches Patentamt

European Patent Office

Office européen des brevets

(11) Publication number: **0 305 470 B1**

## (12) EUROPEAN PATENT SPECIFICATION

(45) Date of publication of patent specification: **11.11.92**  (51) Int. Cl.⁵: **A61K 31/77**

(21) Application number: **88902681.1**

(22) Date of filing: **19.02.88**

(86) International application number:
**PCT/US88/00510**

(87) International publication number:
**WO 88/06038 (25.08.88 88/19)**

(54) ANTIINFECTIVE COMPOUNDS AND METHOD OF USE.

| | |
|---|---|
| (30) Priority: **20.02.87 US 17330**<br>**16.02.88 US 150731** | (73) Proprietor: **EMORY UNIVERSITY**<br>**1380 South Oxford Road**<br>**Atlanta, GA 30322(US)** |
| (43) Date of publication of application:<br>**08.03.89 Bulletin 89/10** | (72) Inventor: **HUNTER, Robert, L.**<br>**3640 Churchwell Court**<br>**Tucker, GA 30084(US)** |
| (45) Publication of the grant of the patent:<br>**11.11.92 Bulletin 92/46** | |
| (84) Designated Contracting States:<br>**AT BE CH DE FR GB IT LI LU NL SE** | (74) Representative: **Sternagel, Hans-Günther, Dr.**<br>**et al**<br>**Patentanwälte Dr. Michael Hann Dr. H.-G.**<br>**Sternagel Sander Aue 30**<br>**W-5060 Bergisch Gladbach 2(DE)** |
| (56) References cited:<br>**EP-A- 0 000 704**<br>**EP-A- 0 049 422**<br>**EP-A- 0 103 290**<br>**FR-A-20 814 36**<br>**US-A-31 402 32** | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The present invention relates to the use of specific antiinfective compounds and more particularly, to compounds that kill or suppress the growth of bacteria and viruses.

FR-A-2081436 discloses an orally administrable pharmaceutical composition comprising a nonionic block copolymer and a tetracycline, chloramphenicol or griseofulvine wherein the copolymer acts as a non-antichlolinergic and antispasmodic agent which reduces the normal mobility or peristaltic action of the gastro-intestinal tract.

From US-A-3,140,232 a pharmaceutical composition is known comprising a tetracycline antibiotic as active ingredient and polypropylene glycol as carrier and stabilizer.

The term "strain" as used in the present invention means any genetically related organism.

Tuberculosis has been a major killing disease of mankind for most of recorded history. The incidence of the disease has declined in parallel with advancing standards of living since at least the mid-nineteenth century. However, the disease continues to be a major problem in many third world countries.

Tuberculosis characteristically affects poor, malnourished, aged, stressed or immunosuppressed individuals. Recently, public health officials have reported an alarming increase in tuberculosis in two groups of people in the United States. The first are elderly people in nursing homes who have a high incidence of primary tuberculosis. Antibiotic-resistant organisms are common in these patients. With the advancing age of our population, it is feared that tuberculosis among the elderly will become an increasingly severe problem. The second group consists of patients with Acquired Auto Immune Deficiency Disease or AIDS. The individuals in both groups have an increased incidence of infection with *Mycobacterium avium*, a microorganism that is generally resistant to conventional antibiotics.

Mycobacteria, including *Mycobacterium avium*, are intracellular parasites that are capable of growth within cells in the host such as macrophages. The mycobacteria grow slowly, produce no endotoxin and are not motile. They multiply within the macrophages, kill the macrophage and are taken up by new macrophages to start the process over. Host resistance depends upon activation of the macrophages. Activated macrophages are able to kill the bacteria that reside within the cell. This activation depends upon specific T-cells which are produced as the result of a cell mediated immune reaction against proteins of the mycobacteria. Mycobacterial infections have been likened to a war of attrition in which there is a delicate balance between the ability of the mycobacteria to survive within the macrophages and the ability of the host to activate macrophages sufficiently to kill them. In the absence of rapidly acting antiinfective compounds, the goal of therapy is to tilt the balance in favor of the host.

There is still no clear understanding of the factors which contribute to the virulence of mycobacteria. Many investigators have implicated lipids of the cell wall and bacterial surface as contributors to colony morphology and virulence. Evidence suggests that C-mycosides, on the surface of certain mycobacterial cells, are important in facilitating survival of the organism within macrophages.

*Mycobacterium avium* bacilli occur in several distinct colony forms. Bacilli which grow as transparent or rough colonies on conventional laboratory media are able to multiply within macrophages in tissue culture, are virulent when injected into susceptible mice, and are resistant to antibiotics. Rough or transparent bacilli which are maintained on laboratory culture media often spontaneously assume an opaque colony morphology at which time they fail to grow in macrophages, are avirulent in mice, and are highly susceptible to antibiotics. The differences in colony morphology between the transparent, rough and opaque strains of *Mycobacterium avium* are almost certainly due to the presence of a glycolipid coating on the surface of transparent and rough organisms which acts as a protective capsule. This capsule, or coating, is composed primarily of C-mycosides which apparently shield the virulent *Mycobacterium avium* organisms from lysosomal enzymes and antibiotics. By contrast, the non-virulent opaque forms of *Mycobacterium avium* have very little C-mycoside on their surface. Both resistance to antibiotics and resistance to killing by macrophages have been attributed to the glycolipid barrier on the surface of *Mycobacterium avium*.

Fatty acids have been shown to exert either an inhibitory or enhancing effect on mycobacterial growth depending on the length of the carbon chain, the degree of saturation, and the presence and type of hydrophilic groups. The mechanism of the growth inhibition effect of fatty acids is not understood, but they are known to be surface-active agents which can interact with glycolipids on the surface of the mycobacteria.

Cornforth *et al*. ("Antituberculosis Effect of Certain Surface-Active Polyoxyethylene Ethers in Mice." *Nature*, 168; 150-153, 1951) reported that certain nonionic surface active agents have antituberculosis effects in mice. Effective materials were arylalkyl polyether ethoxylates, Triton® WR1339 and Triton® A20. These agents are polymerized forms of familiar Triton surfactants. Mice injected intravenously with 15-25 milligrams of the material were protected from lethal challenge with virulent *Mycobacterium tuberculosis* organisms. The material could be injected before, or up to five days after injection of the organisms with mycobacteria. The untreated animals died within three weeks after injection. Over 80% of the treated animals survived at least three weeks with evidence of only minimal tuberculosis lesions.

Triton® WR1339 and Triton® A20 are mixtures of numerous similar compounds and are toxic. Cornforth and his colleagues synthesized a number of similar materials having greater purity, and found that the antituberculosis effect increased with the molecular weight of the hydrophobic portion of the molecules. The most effective pure preparation had four alkylphenol groups attached in a ring configuration. The size of the hydrophilic moiety was also important. Preparations with an average of 15 to 20 polyoxyethylene (POE) moieties per phenolic group were most effective in treating tuberculosis. However, increasing the number of POE moieties to 60 produced a compound which caused infection to progress more rapidly than untreated controls.

Numerous studies were done to evaluate the mechanisms of the tuberculosis and antituberculosis effects of these compounds. The antituberculosis compounds were found to impede the growth of virulent tuberculosis bacteria in intact animals and in macrophages in tissue culture. However, they had no effect on the growth of the organisms in bacterial culture. Consequently, the agents affected either the host or the host-parasite interactions, but had no direct effect on mycobacteria. The bulk of evidence suggested that the tuberculosis and antituberculosis effects were due to modification of surface lipids of the mycobacteria.

The purified Cornforth compounds were not developed commercially as antimycobacterial agents. The reasons for this decision are not known, but several factors may have contributed. First, none of the preparations were pure. Second, the compounds suppressed the growth of mycobacteria in animals but did not produce cures. Finally, the compounds were found to be significantly toxic, producing disorders such as necrosis of the liver.

It is known that nonionic surfactants, in general, are much less toxic than either anionic or cationic surfactants. The nonionic block copolymers are among the least toxic of known surfactants. Nonionic block copolymer surfactants can be synthesized in forms which span virtually the entire range of physical chemical activities of known nonionic surface active agents. Wetting agents with properties reminiscent of Cornforth's antituberculosis agents can be produced in many molecular configurations. They can be produced in a higher molecular weight and in a purer form than is practically feasible with most other surfactants. Problems with toxicity of inactive contaminants can be minimized. The known effects of the block copolymers on serum lipids suggest that the block copolymers have biologic activities similar to those of the agents studied by Cornforth with less toxic effects.

Consequently, there is an immediate and increasing need for a new, safe and effective compound that will have an appropriate effect on mycobacteria organisms present in macrophages which does not exhibit excessive toxicity.

Acquired Immune Deficiency Syndrome, or AIDS, is a disease thought to be caused by a human retrovirus, the Human T Lymphotropic Virus III (HTLV-III) which is also called human immunodeficiency virus or HIV. Like other retroviruses, HTLV-III has ribonucleic acid, or RNA, as its genetic material. When the virus enters the host cell, a viral enzyme called reverse transcriptase exploits the viral RNA as a template to assemble a corresponding molecule of DNA. The DNA travels through the cell nucleus and inserts itself among the host chromosomes, where it provides the basis for viral replication.

In the case of HTLV-III, the host cell is often a T4 lymphocyte, a white blood cell that has a central and regulatory role in the immune system. Once it is inside a T4 cell, the virus may remain latent until the lymphocyte is immunologically stimulated by a secondary infection. Then the virus bursts into action, reproducing itself rapidly so that the new virus particles escaping from the cell riddle the cellular membrane with holes, and the lymphocyte dies. The resulting depletion of the T4 cells leaves the patient vulnerable to "opportunistic" infections by an agent that would not normally harm a healthy person.

There are several therapies against AIDS infection that are currently being investigated. Several of these therapies currently under investigation are based on interrupting the reverse transcriptase as it assembles the viral DNA destined to become the virus. The drugs used for this purpose are chemical analogs of the nucleic acids that form the subunits of DNA. When the analog is supplied to an infected cell, reverse transcriptase will incorporate it into a growing DNA chain. Because the analog lacks the correct attachment point for the next subunit, however, the chain is terminated. The truncated DNA cannot integrate itself into the host chromosomes or provide the basis for viral replication, and so the spread of the infection is halted. One of the compounds that is thought to act by mimicking a nucleotide is azidothymidine, or AZT. However, AZT is known to have serious side effects and its efficacy in mitigating the AIDS disease has been questioned.

Consequently, there is an immediate need for a compound that will suppress or halt the replication and infection of cells by the viruses such as the HTLV-III virus.

3

It is the object of the invention to provide a pharmaceutical composition for the therapy of a human or animal against infections.

The object of the invention is attained by the use of nonionic block copolymer having the following general formula:

$$HO(C_2H_4O)_b(C_3H_6O)_a(C_2H_4O)_bH$$

wherein

i. a is an integer such that the molecular weight represented by the polyoxypropylene portion of the copolymer is between 1,200 and 4,000; and

ii. b is an integer such that the molecular weight represented by the polyoxyethylene portion of the copolymer constitutes between 10 percent and 50 percent of the copolymer

for the manufacture of an injectable pharmaceutical composition for the therapy of a human or animal against infections.

The bactericidal composition prepared by the use of the present invention is usually administered by intravenous injection into a patient.

The present invention provides a composition that can be administered to patients who are infected with *Mycobacterium species*. The surface active copolymer is effective in inhibiting the growth of *Mycobacterium species* and also causes the bacterium to be more susceptible to conventional antibiotics.

Accordingly, the manufactured composition can be used to treat persons with bacterial diseases such as tuberculosis and for treating viral infections in humans or animals.

The composition is effective in inhibiting the replication of viruses in both animals and humans and in inhibiting the replication of the HTLV-III virus or related virus strains in humans.

An object of the present invention is to provide a surfactant compound that can be used to treat mycobacterial infections in persons with AIDS.

The composition can be used to prevent the development of tuberculosis in elderly persons.

The composition will inhibit the growth of *Mycobacterium avium*.

The surfactant compound causes the *Mycobacterium species* to be more susceptible to conventional antibiotics.

These features and advantages of the present invention will become apparent after a review of the following detailed description of the disclosed embodiment and the appended claims.

## Brief Description of the Drawings

Fig. 1 is a graphical representation of the effect of L101 on the growth of *Mycobacterium avium*.

Fig. 2 is a graphical representation of the effect of L105 on the growth of *Mycobacterium avium*.

Fig. 3 is a graphical representation of the effect of L103 on the growth of *Mycobacterium avium*.

Fig. 4 is a graphical representation of the effect of L108 on the growth of *Mycobacterium avium*.

Fig. 5 is a graphical representation of the effect of 11 non-ionic copolymers on the growth of *Mycobacterium avium*.

Fig. 6 is a graphical representation of the effect of several non-ionic copolymer on *Mycobacterium avium* growth.

Fig. 7 is a graphical representation of the effect of the P103 copolymer on HTLV-III virus infection in H9 cells.

The use according to the present invention provides a composition which inhibits the growth of bacteria and viruses. An example of the bacteria that the composition is effective against is *mycobacteria species*.

The composition can be used for treating a viral infection in a human or animal including infections caused by the HLTV-III virus or related strains. The present invention provides a composition that can be administered to patients who are infected with HTLV-III viruses or similar viruses. The surface-active copolymer is effective in inhibiting or suppressing the replication of the HTLV-III virus and related virus strains in cells. The present invention also includes a composition useful for treating mycobacterial infections comprising a mixture of a nonionic block copolymer and selected antibiotics. Several conventional antibiotics that can be used with the nonionic copolymer include, but are not limited to, refampin, isoniazid, ethambutol, gentamicin and erythromycin.

The present invention comprises a surface active copolymer that is an ethylene oxide-propylene oxide condensation product with the following general formula:

$$HO(C_2H_4O)_b(C_3H_6O)_a(C_2H_4O)_bH$$

wherein a is an integer such that the hydrophobe represented by ($C_3H_6O$) has a molecular weight of 1200 to 4000, preferably 1750 to 3500, and b is an integer such that the hydrophile portion represented by ($C_2H_4O$) constitutes 10% to 50% by weight of the compound.

The entire molecule is poorly soluble in water and is either a nonionic or a weakly cationic surfactant. The steric configurations and physiochemical properties of the molecule, rather than the chemical nature of the constituent parts, are believed to be largely responsible for the antiinfective activity.

The polymer blocks are formed by condensation of ethylene oxide and propylene oxide, at elevated temperature and pressure, in the presence of a basic catalyst. There is some statistical variation in the number of monomer units which combine to form a polymer chain in each copolymer. The molecular weights given are approximations of the average weight of copolymer molecule in each preparation. It is to be understood that the blocks of propylene oxide and ethylene oxide do not have to be pure. Small amounts of other materials can be admixed so long as the overall physical chemical properties are not substantially changed. A more detailed discussion of the preparation of these products is found in US -A- 2,674,619.

Ethylene oxide-propylene oxide condensation products which may be employed in the present invention are summarized in Table I.

Table I

| Copolymer | Molecular Weight of Polyoxypropylene | % Polyoxyethylene |
|---|---|---|
| L42 | 1200 | 20% |
| L61 | 1750 | 10% |
| L62 | 1750 | 20% |
| L63 | 1750 | 30% |
| L64 | 1750 | 40% |
| P65 | 1750 | 50% |
| F68 | 1750 | 80% |
| L72 | 2050 | 20% |
| L81 | 2250 | 10% |
| L92 | 2750 | 20% |
| L101 | 3250 | 10% |
| P103 | 3250 | 30% |
| P105 | 3250 | 50% |
| F108 | 3250 | 80% |
| L121 | 4000 | 10% |
| L122 | 4000 | 20% |
| P123 | 4000 | 30% |

The polymer blocks are formed by condensation, at elevated temperature and pressure, of ethylene oxide and propylene oxide in the presence of a basic catalyst. There is some statistical variation in the number of monomer units which combine to form a polymer chain in each copolymer. The molecular weights given are approximations of the average size of copolymer molecules in each preparation. A further description of the preparation of these block copolymers is found in US -A- 2,674,619. (Also see, "A Review of Block Polymer Surfactants", Schmolka I.R., *J. Am. Oil Chemist Soc.*, 54:110-116 (1977) and *Block and Graft Copolymerization*, Volume 2, edited by R.J. Ceresa, John Wiley and Sons, New York, 1976.

The preferred ethylene oxide-propylene oxide copolymer for use according to the present invention is a copolymer having the following formula:

$$HO(C_2H_4O)_b(C_3H_6O)_a(C_2H_4O)_bH$$

wherein a is an integer such that the hydrophobe represented by ($C_3H_6O$) has a molecular weight of 2500 to 3800, preferably 1750 to 4000, and b is an integer such that the hydrophile portion represented by ($C_2H_4O$) constitutes 10% to 40% by weight of the compound.

An especially preferred embodiment of the antiinfective compound is the compound designated P103 with the following general formula:

$HO(C_2H_4O)_b(C_3H_6O)_a(C_2H_4O)_bH$

wherein the mean aggregate molecular weight of the hydrophobic portion of the triblock copolymer, consisting of polyoxypropylene is 3,250 daltons; a is an integer such that the total molecular weight of the polyoxypropylene (POP) portion of the triblock copolymer constitutes 70% of the compound; and b is an integer such that the hydrophile portion represented by polyoxyethylene (POE) constitutes 10% of the total molecular weight of the compound.

The antiinfective compound is effective in suppressing the growth of *Mycobacterium avium* in humans suffering from a variety of health disorders. The disorders in which the antiinfective compound is infective include, but are not limited to, tuberculosis, cancer and AIDS.

The antiinfective composition prepared according to the present invention is effective with only one injection of compound being administered to the patient. However, in certain cases, subsequent injections may be necessary to achieve maximum efficiency. The mode of injection can be subcutaneous, intramuscular or intravenous. The preferred mode of injection is subcutaneous.

The optimum amount of the antiinfective compound in an injection varies with the weight of the patient being treated. The effective dose rate appears to be in the range of $1 \times 10^{-3}$ M to $1 \times 10^{-4}$ M (approximately 1.5mg/ml).

The following specific examples will illustrate the invention as it applies in particular to *in vitro* suppression of the growth of colonies of *Mycobacterium avium* and HTLV III virus *in vitro* isolated from humans. It will be appreciated that other examples will be apparent to those skilled in the art and that the invention is not limited to these specific illustrative examples

**Example 1**

Smooth transparent (virulent) and smooth domed (non-virulent) colonies of *Mycobacterium avium* derived from the same patient isolate were grown m 7H12 Middlebrook broth (Johnston Laboratories, Cockeysville, MD). The Bactec 7H12 Middlebrook TB medium is an enriched Middlebrook 7H9 broth base which has been supplemented with bovine serum albumin (fraction V), catalase, casein hydrolysate and $C^{14}$-labeled fatty acids. Mycobacteria metabolize the $C^{14}$-labeled substrates and release $CO_2$ in air, thereby maintaining the recommended atmosphere. In addition, vials were inoculated with antimycobacterial agents and M. tuberculosis so that the evolution of $^{14}CO_2$ can be correlated to the susceptibility or resistance of the organism to the drug. When the mycobacteria grow in the medium containing 14C-labeled fatty acid, they utilize the fatty acid and $^{14}CO_2$ is produced. The production of $CO_2$ can be detected quantitatively, reflecting the rate and amount of growth occurring in the vial, and is expressed in terms of the "growth index". If an anti-tuberculosis drug is added to the medium, suppression of growth occurs in the case of susceptible organisms which can be detected by either a decline or a very small increase of the growth index as compared to the control. However, if the organisms are resistant, no suppression occurs in the rate of increase of the growth index on daily testing.

To determine the 1% proportion of resistance, the inoculum in the control vial is one hundred fold less than the inoculum used for drug containing vials. Growth index readings are taken each day after inoculation and the increase in growth index over that of the previous day, is compared for the control vial and the vials containing drugs. If the daily increase in growth index, called delta growth index, in the drug vial is equal to or greater than that in the control vial, the test organisms are considered resistant to the drug. For a susceptible organism, the daily increase in the growth index for the control would be much higher than for the drug vial.

Cultures were incubated at 36° C and checked daily with a Bactec 460TB (Johnston Laboratories, Cockeysville, MD) instrument with a self-contained laminar flow hood. A growth index was determined by measuring the amount of $C^{14}$ released into the atmosphere of the container as a result of mycobacterial utilization of $C^{14}$-labeled fatty acids.

*Mycobacterium avium* organisms were treated with one of four nonionic block copolymers, designated L101, P103, P105, and F108. These copolymers have identical hydrophobic portions and differ only in the length of the polyoxyethylene (hydrophilic) chains. These four molecules range in size from 3,600 to 14,000 daltons and the molecular weight attributable to the polyoxypropylene portion of each molecule is 3,250. The hydrophilic portion of each molecule is 10% for L101, 30% for P103, 50% for P105, and 80% for F108. Each of the candidate anti-mycobacterial copolymers were mixed with the Middlebrook tuberculosis medium at concentration of $1 \times 10^{-3}$ M and $1 \times 10^{-4}$ M.

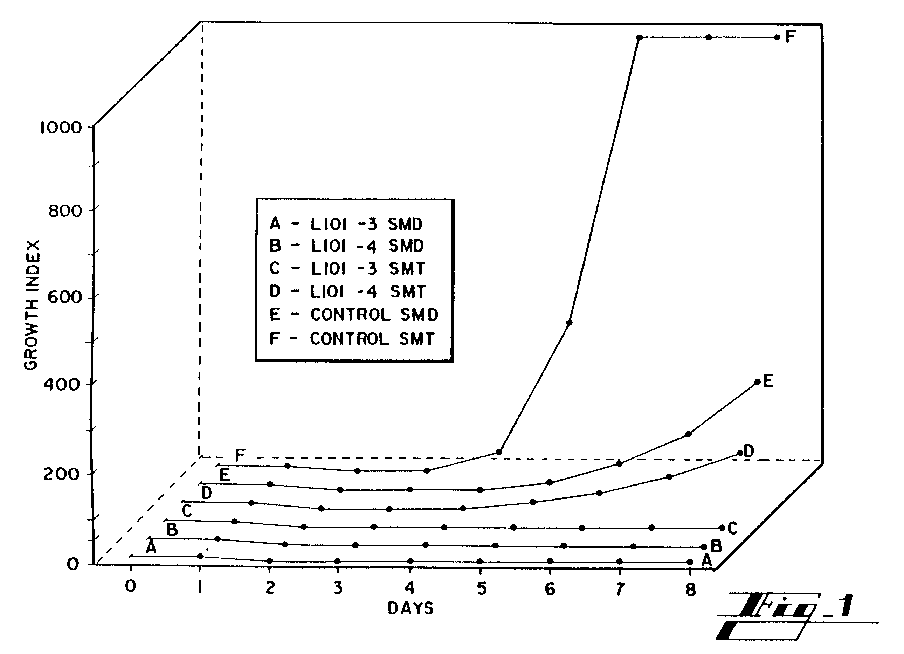

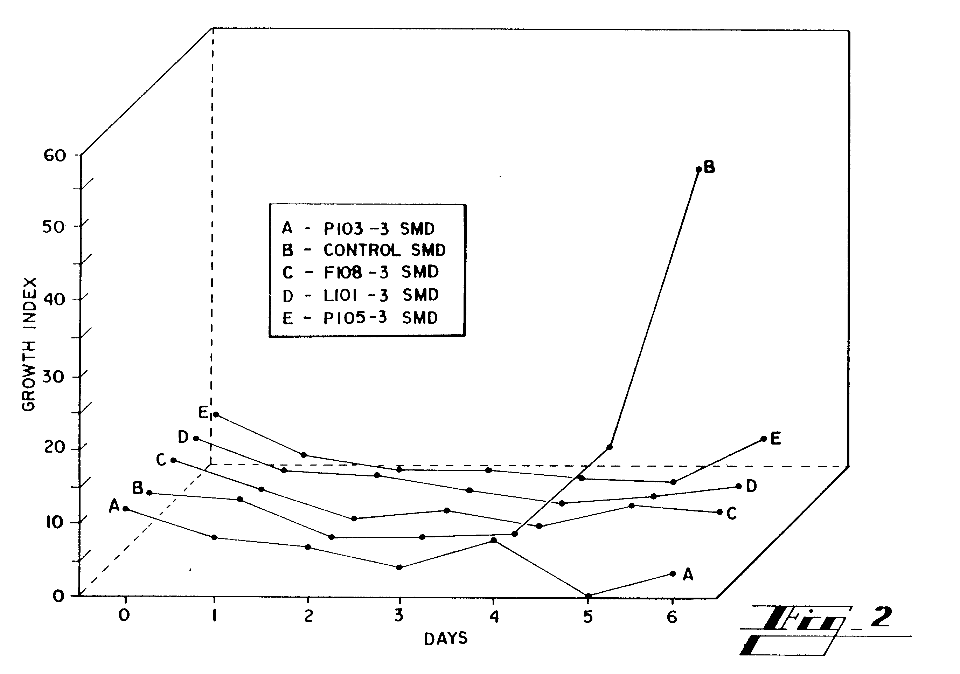

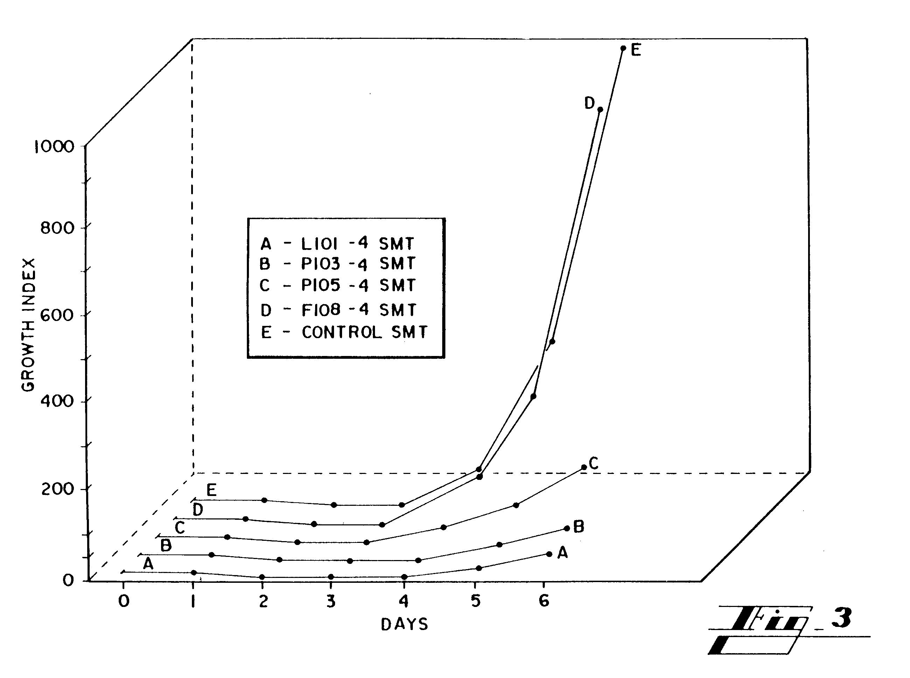

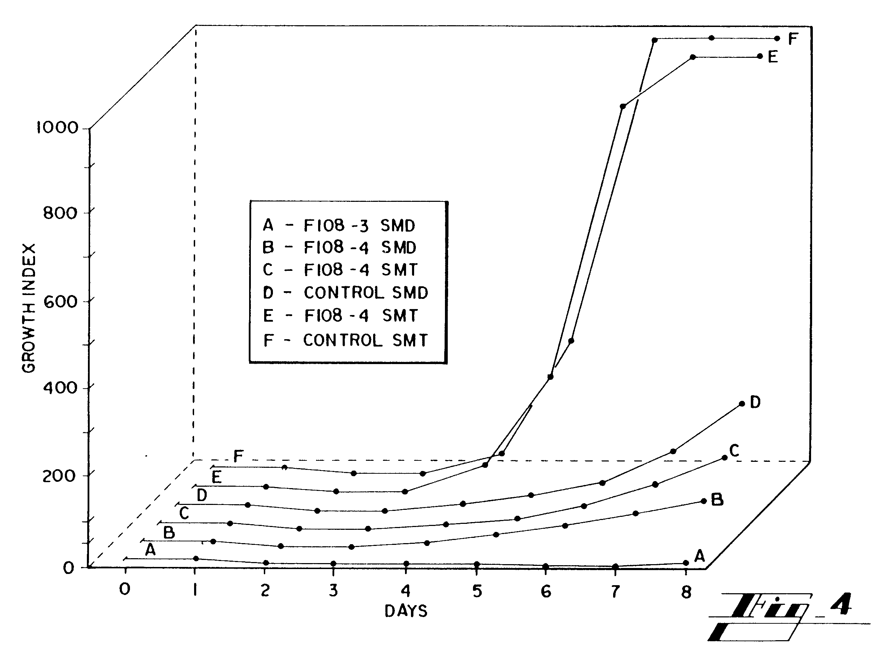

As shown in Figs. 1, 2, and 3, a significant reduction in growth rate of both smooth transparent (SmT) colonies treated with L101, P103, and P105. As shown in Fig. 4, smooth transparent (virulent) colonies treated with F108 in a concentration of $1 \times 10^{-4}$ M did not inhibit the growth of *Mycobacterium avium*; however, an F108 concentration of $1 \times 10^{-3}$ M showed some suppression of bacterial growth.

**Example 2**

Smooth transparent (virulent) and smooth domed (nonvirulent) colonies of *Mycobacterium avium* derived from the same patient isolate were grown in BACTEC 7H12 Middlebrook broth. A growth index was determined by measuring the amount of $C^{14}$ released into the atmosphere of the container as a result of mycobacterial utilization of $C^{14}$-labeled fatty acid as described in Example I.

*Mycobacterium avium* organisms treated with one of fourteen nonionic block copolymers, designated L42, L61, L62, L63, L64, P65, L72, L81, L92, L101, P103, L121, L122, and P123. Each copolymer has a polyoxyethylene portion ranging from 10% to 50% of the total molecule. The molecular weight of the polyoxypropylene portion of each molecule ranged from 1,200 to 4,000. The physical characteristics of each copolymer are summarized in Table I.

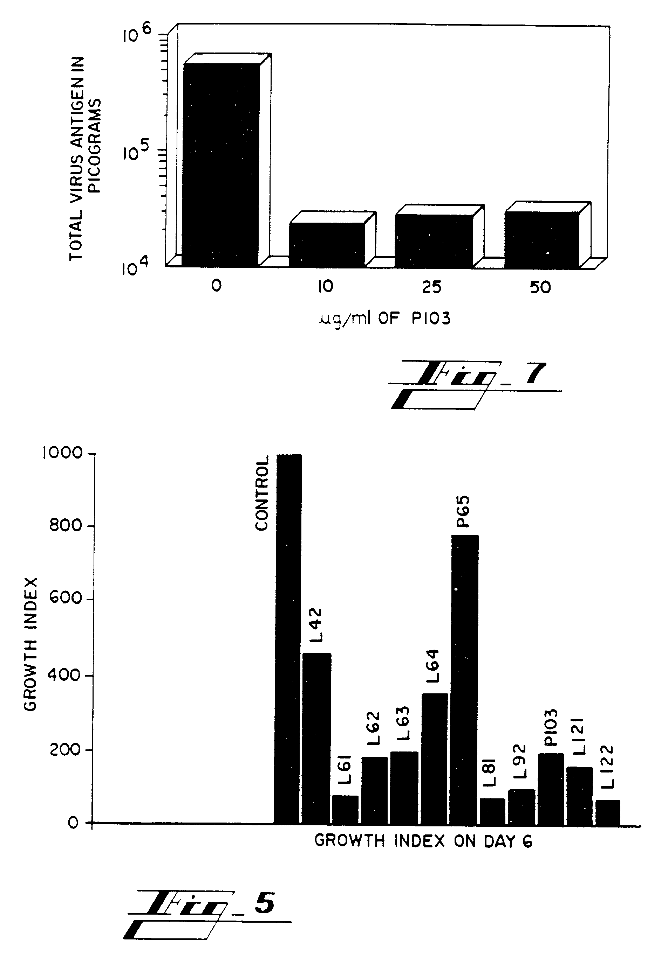

As shown in Fig. 5, there was a significant reduction in growth rate of smooth transparent colonies treated with all the copolymers tested. P65 and L42 exhibited only moderate inhibition.

**Example III**

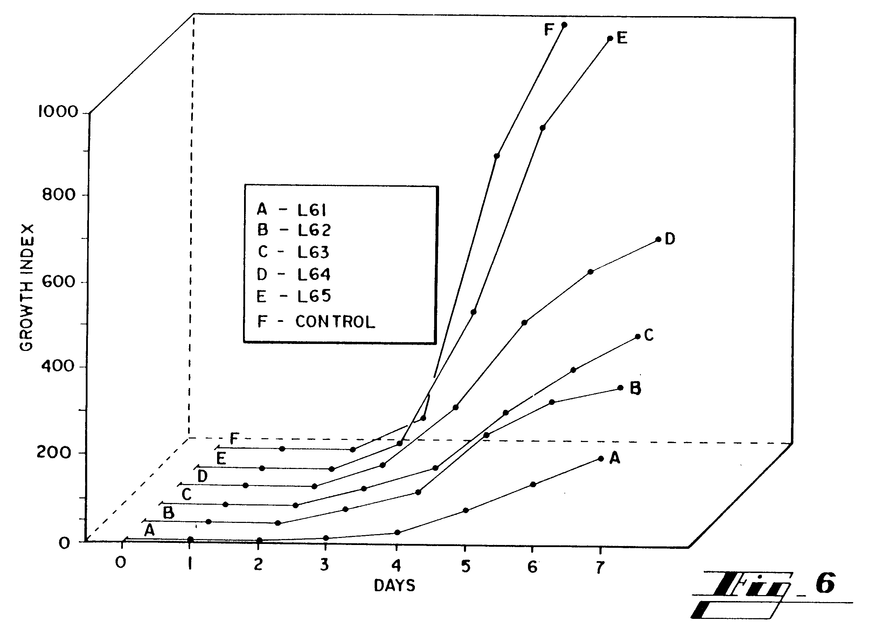

A correlation between increasing hydrophobicity of the copolymer and inhibition of *M. avium* growth is shown in Fig. 6. The copolymers having a polyoxypropylene molecular weight of 1750 were tested for inhibition of *M. avium* . The copolymers tested are summarized in the following Table II:

Table II

| Copolymer | Molecular Weight of Polyoxypropylene | % Polyoxyethylene |
|---|---|---|
| L61 | 1750 | 10% |
| L62 | 1750 | 20% |
| L63 | 1750 | 30% |
| L64 | 1750 | 40% |
| P65 | 1750 | 50% |

As shown in Fig. 6, inhibition of mycobacterial growth specifically correlates with the molecular structure of the copolymers. The low molecular weight copolymers which have only a small percentage of hydrophil appear to inhibit *M. avium* growth more than copolymers which are more hydrophilic.

**Example IV**

The effectiveness of P103 copolymer is examined in preventing HTLV-III replication in H9 cells using a high multiplicity of viral infection. The infections are conducted in the following manner: $10^5$ H9 cells in 1 ml medium containing $1.89 \times 10^{-2}$ picogram T24 viral antigen per cell, and either 0, 10, 25, or 50 $\mu$g of P103, are incubated at 37°C in 5% $CO_2$ for one hour. After this time, the cells are pelleted at 3,000 rpm and washed to remove access non-adsorbed virus. The cells are plated in medium in dishes containing wells of 1 cm diameter. The cultures and the controls, all done in triplicate, are incubated at 37°C, 5% $CO_2$, and the volume in each well is maintained at a constant volume of 1 ml. The media contains either 10, 25, or 50 $\mu$g per ml of P103. Virus yields in terms of total picogram of T24 HIV antigen and cell counts are determined on day seven of the incubation period. Cell viability is determined by trypan blue exclusion.

As can be seen in Figure 7, there is at least a one and one/half log reduction in virus yield already evident at the low concentration of 10 $\mu$g per ml of P103. Furthermore, the higher concentrations of P103 did not appear to mediate further reductions in viral replication.

According to these results, the P103 copolymer is considered an effective inhibitor of HIV application in H9 cells.

**Claims**

1. Use of nonionic block copolymer having the following general formula:

$$HO(C_2H_4O)_b(C_3H_6O)_a(C_2H_4O)_bH$$

wherein
 i. a is an integer such that the molecular weight represented by the polyoxypropylene portion of the copolymer is between 1,200 and 4,000; and
 ii. b is an integer such that The molecular weight represented by the polyoxyethylene portion of the copolymer constitutes between 10 percent and 50 percent of the copolymer
for the manufacture of an injectable pharmaceutical composition for the therapy of a human or animal against infections.

2. The use of the nonionic block copolymer of claim 1 for the manufacture of an injectable pharmaceutical composition for the therapy of a human or animal against bacterial infections.

3. The use of the nonionic block copolymer of claims 1 or 2,
**characterized in that**
the bacterial infections are caused by a Mycobacterium species.

4. The use of the nonionic block copolymer of claim 1 for the manufacture of an injectable pharmaceutical composition for the therapy of a human or animal against viral infections.

5. The use of the non-ionic block copolymer of claim 4
**characterized in that**
the viral infections are caused by a virus which is a HLTV-III virus or antigenically related virus strains.

6. The use of the nonionic block copolymer of claim 1
**characterized in that**
the molecular weight represented by the polyoxypropylene portion of the copolymer is between 1750 to 3500.

7. The use of the nonionic block copolymer of claim 1
**characterized in that**
the molecular weight represented by the polyoxypropylene portion of the copolymer is 3250 and the molecular weight represented by the polyethylene portion of the copolymer constitutes 10 percent of the copolymer.

8. The use of the nonionic block copolymer of any of claims 1 to 7 for the manufacture of subcutaneous injectable pharmaceutical composition.

9. The use of the nonionic block copolymer of any of claims 1 to 7 for the manufacture of intramuscular injectable pharmaceutical composition.

10. The use of the nonionic block copolymer of any of claims 1 to 7 for the manufacture of intravenous injectable pharmaceutical composition.

11. The use of the nonionic block copolymer of any of claims 1 or 10
**characterized in that**
said copolymer is used in combination with antibiotics selected from rifampin, isoniazid, ethambutol, gentamicin and erythromycin.

**Patentansprüche**

1. Verwendung nichtionischer Blockcopolymerer der nachfolgenden allgemeinen Formel:

$$HO(C_2H_4O)_b(C_3H_6O)_a(C_2H_4O)_bH,$$

in der

EP 0 305 470 B1

i. a eine solche ganze Zahl ist, daß das Molekulargewicht des Polyoxypropylenteils des Copolymeren zwischen 1.200 und 4.000 beträgt, und

ii. b eine solche ganze Zahl ist, daß das Molekulargewicht des Polyoxyethylenteils des Copolymeren zwischen 10 und 50% des Copolymeren ausmacht,

zur Herstellung einer injizierbaren pharmazeutischen Zusammensetzung zur Behandlung eines Menschen oder Tieres gegen Infektionen.

2. Verwendung des nichtionischen Blockcopolymeren nach Anspruch 1 zur Herstellung einer injizierbaren pharmazeutischen Zusammensetzung zur Behandlung eines Menschen oder Tieres gegen bakterielle Infektionen.

3. Verwendung des nichtionischen Blockcopolymeren nach Ansprüchen 1 oder 2,
**dadurch gekennzeichnet**,
daß die bakteriellen Infektionen durch Mycobakteriumspezies verursacht sind.

4. Verwendung des nichtionischen Blockcopolymeren nach Anspruch 1 zur Herstellung einer injizierbaren pharmazeutischen Zusammensetzung zur Behandlung eines Menschen oder Tieres gegen Virusinfektionen.

5. Verwendung des nichtionischen Blockcopolymeren von Anspruch 4,
**dadurch gekennzeichnet**,
daß die Virusinfektionen durch einen Virus, der ein HLTV-III Virus ist, oder einen antigenisch verwandten Virusstamm ausgelöst sind.

6. Verwendung des nichtionischen Blockcopolymeren nach Anspruch 1,
**dadurch gekennzeichnet**,
daß das Molekulargewicht des Polyoxypropylenanteils des Copolymeren zwischen 1750 und 3500 beträgt.

7. Verwendung des nichtionischen Blockcopolymeren nach Anspruch 1,
**dadurch gekennzeichnet**,
daß das Molekulargewicht des Polyoxypropylenanteils des Copolymeren 3250 ist und das Molekulargewicht des Polyethylenanteils des Copolymeren 10% des Copolymeren ausmacht.

8. Verwendung des nichtionischen Blockcopolymeren nach jedem der Ansprüche 1-7 zur Herstellung einer unter die Haut injizierbaren pharmazeutischen Zusammensetzung.

9. Verwendung des nichtionischen Blockcopolymeren nach jedem der Ansprüche 1-7 zur Herstellung einer intramuskulär injizierbaren pharmazeutischen Zusammensetzung.

10. Verwendung des nichtionischen Blockcopolymeren nach jedem der Ansprüche 1-7 zur Herstellung einer intravenös injizierbaren pharmazeutischen Zusammensetzung.

11. Verwendung des nichtionischen Blockcopolymeren nach jedem der Ansprüche 1-10,
**dadurch gekennzeichnet**,
daß das Copolymere in Kombination mit Antibiotika verwendet wird, ausgewählt aus Rifampin, Isoniazid, Ethambutol, Gentamicin und Erythromycin.

**Revendications**

1. Utilisation de copolymères bloc non ioniques ayant la formule générale suivante :

$$HO(C_2H_4O)_B(C_3H_6O)_a(C_2H_4O)_bH$$

dans laquelle.

i. a est un nombre entier tel que le poids moléculaire représenté par la fraction polyoxypropylène du copolymère est compris entre 1200 et 4000 ; et

ii. b est un nombre entier tel que le poids moléculaire représenté par la fraction polyoxyéthylène du

9

copolymère constitue entre 10 et 50 % du copolymère.
pour la fabrication d'une composition pharmaceutique injectable en vue du traitement de l'homme et de l'animal contre les infections.

2. Utilisation du copolymère bloc non ionique de la revendication 1 pour la fabrication d'une composition pharmaceutique injectable en vue du traitement de l'homme et de l'animal contre les infection bactériennes.

3. Utilisation du copolymère bloc non ionique de la revendication 1 ou 2, caractérisée en ce que les infections bactériennes sont causées par une souche de Mycobactérium.

4. Utilisation du copolymère bloc non ionique de la revendication 1 pour la fabrication d'une composition pharmaceutique injectable pour le traitement de l'homme ou de l'animal contre les infections virales.

5. Utilisation du copolymère bloc non ionique de la revendication 4, caractérisée en ce que les infections virales sont causées par un virus qui est HLTV-III ou des souches de virus apparentées antigénétiquement.

6. Utilisation du copolymère bloc non ionique de la revendication 1, caractérisée en ce que le poids moléculaire représenté par la fraction polyoxypropylène du copolymère est compris entre 1750 et 3500.

7. Utilisation du copolymère bloc non ionique de la revendication 1, caractérisée en ce que le poids moléculaire représenté par la fraction polyoxypropylène du copolymère est 3250 et le poids moléculaire représenté par la fraction polyéthylène du copolymère constitue 10 % du copolymère.

8. Utilisation du copolymère bloc non ionique selon l'une quelconque des revendications 1 à 7 pour la fabrication d'une composition pharmaceutique injectable par voie sous-cutanée.

9. Utilisation du copolymère bloc non ionique selon l'une quelconque des revendications 1 à 7 pour la fabrication d'une composition pharmaceutique injectable par voie intramusculaire.

10. Utilisation du copolymère bloc non ionique selon l'une quelconque des revendications 1 à 7, pour la fabrication d'une composition pharmaceutique injectable par voie intraveineuse.

11. Utilisation du copolymère bloc non ionique selon l'une quelconque des revendications 1 à 10, caractérisée en ce que le dit polymère est utilisé en combinaison avec des antibiotiques choisis parmi la rifampicine, l'isoniazide, l'éthambutol, la gentamycine et l'érythromycine.

Fig. 1

EP 0 305 470 B1

**Fig. 2**

GROWTH INDEX

60

50

40

30

20

10

0

A - PIO3 -3 SMD
B - CONTROL SMD
C - FIO8 -3 SMD
D - LIOI -3 SMD
E - PIO5-3 SMD

0    1    2    3    4    5    6

DAYS

EP 0 305 470 B1

A – LIOI –4 SMT
B – PIO3 –4 SMT
C – PIO5 –4 SMT
D – FIO8 –4 SMT
E – CONTROL SMT

Fig. 3

EP 0 305 470 B1

GROWTH INDEX

A – F108 –3 SMD
B – F108 –4 SMD
C – F108 –4 SMT
D – CONTROL SMD
E – F108 –4 SMT
F – CONTROL SMT

DAYS

Fig. 4

EP 0 305 470 B1

TOTAL VIRUS ANTIGEN IN PICOGRAMS

$10^6$

$10^5$

$10^4$

0    10    25    50

μg/ml OF PI03

Fig_7

GROWTH INDEX

1000

800

600

400

200

0

CONTROL

L42

L61

L62

L63

L64

P65

L81

L92

PI03

L121

L122

GROWTH INDEX ON DAY 6

Fig_5

15

EP 0 305 470 B1